# EUROPÄISCHE PATENTANMELDUNG

(11) **EP 1 407 760 A1**
(43) Veröffentlichungstag der Anmeldung: **14.04.2004**
(21) Anmeldenummer: 02292475.7
(22) Anmeldetag: 08.10.2002
(51) Int. Cl.: A61K 7/48, A61K 7/06, A61K 31/00, A61K 35/78

(54) **Verfahren zum Schutz der Haut gegen Alterung**

(71) Anmelder: Cognis France S.A., 31360 Saint-Martory (FR)
(72) Erfinder: Pauly, Gilles, 54000 Nancy (FR); Maquart, Francois-Xavier, 51100 Reims (FR); Wegrowski, Yanusz, 51430 Bezannes (FR); Contet-Audonneau, Jean-Luc, 54130 Saint-Max (FR); Danoux, Louis, 54420, Saulxures-Les-Nancy (FR); Jeanmaire, Christine, 54000 Nancy (FR)
(74) Vertreter: Cabinet HERRBURGER

(57) **Zusammenfassung**

Vorgeschlagen wird ein Verfahren zur kosmetischen Behandlung zur Verbesserung und/oder zum Schutz der menschlichen Haut, Kopfhaut und/oder Schleimhaut, dadurch gekennzeichnet, dass man eine Zubereitung, enthaltend mindestens eine Substanz, welche eine Modulation von Proteoglykanen, insbesondere von Lumican und/oder Syndecan und/oder Versican und/oder Decorin und/oder Glypican bewirkt, topisch aufträgt.

Des Weiteren wird die Verwendung einer Substanz, welche eine Modulation von Lumican und/oder Syndecan und/oder Versican und/oder Decorin und/oder Glypican bewirkt, zur Herstellung kosmetischer Mittel zum Schutz gegen Hautalterung, oxidativen Stress, die Verwendung der Substanz zur Herstellung kosmetischer Mittel zum Schutz gegen schädigende Einflüsse durch Umweltgifte und UV-Strahlung, zur Stärkung der Funktionen der Dermal-Epidermal-Junctions und die Verwendung von Zubereitungen zur Verbesserung der Wundheilung und gegen Alopecie, Cellulitis oder Rosacea, vorgeschlagen.

## Beschreibung

Die Erfindung befindet sich auf dem Gebiet der kosmetischen Zubereitungen und betrifft ein kosmetisches Verfahren zum Schutz der menschlichen Haut, Kopfhaut und/oder Schleimhaut gegen die Alterung, oxidativen Stress und gegen schädigende Einflüsse durch Umweltgifte und UV-Strahlung. Des Weiteren betrifft die Erfindung die Verwendung einer Substanz, welche eine Modulation von Lumican und/oder Syndecan und/oder Versican und/oder Decorin und/oder Glypican bewirkt, zur Herstellung kosmetischer Mittel und Mittel gegen besondere Hautkrankheiten.

### Stand der Technik

Die extrazelluläre Matrix des Bindegewebes besteht aus einer Vielzahl von Makromolekülen, die komplexe, dreidimensionale Netzwerke bilden, untereinander und mit den Zellen des Bindegewebes interagieren und so zu der strukturellen Integrität des Gewebes entscheidend beitragen. Sie bildet aber nicht nur ein Bindeglied zwischen einzelnen Zellen und Geweben, sondern sorgt über eine Filter- und Transportfunktion - von Wachstumsfaktoren, Inhibitoren, oder Hormonen - sowie über besondere Rezeptorbindungen auch für die Regulation des Zellwachstums, Versorgung von Zellen und die Zelldifferenzierung. Zu den wesentlichen Bestandteilen der extrazellulären Matrix zählen Collagen, Elastin, Glycoproteine, Hyaluronsäure, Glycosaminoglykane und unterschiedliche Proteoglykane, wobei Proteoglykane, Glucosaminoglykane und Glykoproteine zur nichtfaserigen Grundstruktur gehören.
Die Proteoglykane sind Makromoleküle mit einem Protein im Kern, an das eine oder mehrere Glucosaminoglykan Seitenketten kovalent gebunden sind. Sie stellen die Hauptkomponente in der extrazellulären Matrix dar und beinhalten eine Vielzahl unterschiedlicher Moleküle, die in einer groben Einteilung nach großen und kleinen Proteoglykanen aufgeteilt werden. Proteoglykane befinden sich in allen Ebenen der Basalmembran, die eine weitgehend homogene Schicht dicht unterhalb von den basalen Epithelzellen bildet und sich in drei Schichten unterteilen lässt. Der oberste Teil, der direkt an die Zellen angrenzt, ist die Lamina rara (10-50 nm), als mittlere Schicht der Basalmembran folgt die Lamina densa (20 - 300 nm) und die nach außen liegende Lamina fibroreticularis (200 - 500 nm).
Proteoglykane tragen in der Basalmembran über die Bindungen zu faserigen Gewebsbestandtcilen zur Gewebefestigkeit bei und beeinflussen über die Wasserbindung die Spannkraft der Haut. Untersuchungen haben ergeben, dass die nach hoher UV-Strahlungseinwirkung beobachtete verstärkte Microfältelung der Haut und Abnahme der Elastizität mit einer deutlichen Zunahme des Proteoglykans Versican, dass mit elastischen Fasern assoziiert ist und einer deutlichen Abnahme des Proteoglykans Decorin, das mit Collagenfasern assoziiert ist, korreliert werden konnte.
Während der Alterung erfährt die Haut entscheidende Änderungen in ihren mechanischen Eigenschaften, der Fähigkeit zur Wasserretention , sowie ihrer Spannkraft und Elastizität. Proteoglykane haben hierauf einen entscheidenden Einfluß. Neben der bereits erwähnten Änderung der Proteoglykananteile nach Sonneneinstrahlung ist bei normalen Alterungsprozessen eine gegenläufige Änderung, nämlich mit zunehmendem Alter eine Abnahme der großen Chondroitinsulfat Proteoglycane (Versican) und eine parallele Zunahme der Anteile an kleinen Dermatansulfat Proteoglykanen (Decorin), zu beobachten.
Bekannt ist bisher die Beeinflussung von krankhaften Veränderungen der Haut durch den Eingriff in das System der Proteoglykane. So beschreibt die Internationale Patentanmeldung **WO 01/17560** die Behandlung und Vorbeugung von bakteriellen Infektionen über Substanzen, die die Syndecan-1-Freisetzung hemmen. In der Internationalen Anmeldung **WO 94/12162** wurde die Verminderung des Tumorwachstums und Anregung des Haarwachstums über eine Stimulation von Syndecan offenbart. Es konnte, wie der Patentanmeldung **WO 93/09800** zu entnehmen ist, außerdem gezeigt werden, dass über eine Verabreichung von Decorin, Biglycan und Fibromodulin eine Narbenbildung reduziert oder sogar verhindert wird.

Da neben krankhaften Hautveränderungen auch Bedarf an einer Vorbeugung von alterungsbedingten Hautveränderungen und an einem effektiven Schutz der Haut gegen umweltbedingte Alterungseinflüsse besteht, hat die Aufgabe der vorliegenden Patentanmeldung darin bestanden, neue Mechanismen zur Verbesserung der Haut, Kopfhaut und/oder Schleimhaut zu finden, die zu einer Verzögerung der Hautalterung und zu einem Schutz der Haut, Kopfhaut und/oder Schleimhaut gegen Umwelteinflüsse, oxidativen Stress, toxische Substanzen oder UV-Strahlung beitragen und somit effektiv in kosmetischen und dermopharmazeutischen Zubereitungen für die topische Anwendung genutzt werden können.

### Beschreibung der Erfindung

Gegenstand der Erfindung ist ein Verfahren zur kosmetischen Behandlung zur Verbesserung und/oder zum Schutz der menschlichen Haut, Kopfhaut und/oder Schleimhaut, dadurch gekennzeichnet, dass man eine Zubereitung, enthaltend mindestens eine Substanz, welche eine Modulation von Proteoglykanen, insbesondere von Lumican und/oder Syndecan und/oder Versican und/oder Decorin und/oder Glypican bewirkt, topisch aufträgt.

Weitere Gegenstände der Erfindung sind die Verwendung einer Substanz, welche eine Modulation von Lumican und/oder Syndecan und/oder Versican und/oder Decorin und/oder Glypican bewirkt, zur Herstellung kosmetischer Mittel zum Schutz der menschlichen Haut, Kopfhaut und/oder Schleimhaut gegen Alterung, zur Herstellung kosmetischer Mittel zum Schutz gegen oxidativen Stress, zum Schutz gegen toxische Umwelteinflüsse, zum Schutz gegen Schädigungen durch UV-Lichteinwirkung und zur Verbesserung der Funktionen der Dermal-Epidermal-Junctions, sowie die Verwendung zur Herstellung von dermopharmazeutischen Zubereitungen zur Verbesserung der Wundheilung und Zubereitungen gegen Alopecie, Cellulitis oder Rosacea.
Überraschenderweise wurde gefunden, dass die Modulation von Proteoglykanen, insbesondere von Lumican, Syndecan, Versican, Decorin und/oder Glypican zu einer Verbesserung und zum Schutz der menschlichen Haut, Kopfhaut oder Schleimhaut führt. Es konnte eine Straffung der Haut, eine erhöhte Elastizität und eine bessere Wasserbindungskapazität auch nach Exposition von UV-Strahlung beobachtet werden. Die Regenerationsfähigkeit der Haut ist deutlich verbessert und ermöglicht so, dass die Erneuerung der Haut insbesondere auch zur Wundheilung wesentlich schneller stattfindet und auch bei entzündlichen Hauterkrankungen, insbesondere Alopecie, Cellulitis und Rosacea große Vorteile bringt.
Substanzen, die eine Modulation von Proteoglykanen, insbesondere von Lumican, Syndecan, Versican, Decorin und/oder Glypican bewirken können alleine oder in Kombination mit weiteren Wirkstoffen
- dermale Makromoleküle stärken und resistenter gegen nicht-enzymatische Glykolyse machen und so die Haut gegen toxische Umweltgifte und oxidativen Stress schützen,
- die Balance der Wachstumsfaktoren in der gealterter menschlichen Haut aufrechterhalten, um die Hauterneuerung und Reparatur der Haut nach Schädigungen durch UV-Strahlungseinwirkung oder bei der Wundheilung zu verbessern,
- die Bildung von Mikrofibrillen in der menschlichen Haut unterstützen und somit vor Hautalterungserscheinungen vorbeugen,
- die Funktionen der Dermal-Epidermal Junctions (DEJ) über eine verbesserte Verankerung durch Stärkung der Mikrofibrillen verbessern
- die Wasserbindungskapazität der Haut erhöhen und auf diese Weise zu einer strafferen Haut beitragen,
- die Bildung von Mikrofalten reduzieren und eine weitere Faltenbildung vermindern,
- das Eintreten von Schuppenflechte (Alopecia) verzögern,
- die Hautveränderungen in Folge von Cellulitis und Rosacea reduzieren,
- entzündliche Prozesse, die zu Irritationen, Rötung und Juckreiz führen in ihrer Ausprägung vermindern,
- die Melaninsynthese in der Haut beeinflussen,
- das Immunsystem der Haut stärken und somit das Abwehrsystem gegen schädigende Umwelteinflüsse verbessern.

Als Modulatoren haben sich Pflanzenextrakte, insbesondere der Extrakt aus Pisum sativum und/oder aus Vigna aconitifolia, Extrakte aus Mikroorganismen und/oder fermentationsprodukte pflanzlicher Herkunft als geeignet erwiesen. Möglich ist jedoch auch eine Modulation von Lumican, Syndecan, Versican, Decorin und/oder Glypican über die Applikation von mindestens einer Substanz, die ausgewählt ist aus der Gruppe, die gebildet wird von Mannitol, Cyclodextrin, Hefeextrakt und Dinatriumsuccinat. Insbesondere die Kombination dieser Bestandteile trägt zu einer vorteilhaften Wirkung bei.
Ebenfalls beobachtet wurde eine Modulation der Moleküle über mindestens eine Substanz, die ausgewählt ist aus der Gruppe, die gebildet wird von
- Phytosterolen wie beispielsweise β-Sitosterol, Campestrol, Brassicasterol, Δ5-Avennasterol, α-Spinasterol oder Stigmasterol;
- Phytoestrogenen wie Isoflavonen (Genistein, Daidzein), Stilbenen, Lignan;
- Triterpenen wie Lupeol, Ursolsäure, Arjunoliquesäure, Oleanolic acid;
- Triterpensaponinen und Steroidsaponinen wie Sapogenin, Diosgenin, Hecogenin, Smilagenin, Sarsapogenin, Tigogenin, Yamogenin, Yuccagenin und Bassicsäure;
- Peptiden, insbesondere solchen, die den Wachstumsfaktoren TGFβ, IL4 entsprechen und
- Flavonoiden, sowie Flavonoidderivaten, beobachtet.

Neben diesen Substanzen oder Pflanzenextrakten können die kosmetischen Mittel außerdem UV-Lichtschutzfaktoren und/oder Antioxidantien enthalten. Die Kombination aus Substanzen, die eine Modulation von Lumican und/oder Syndecan und/oder Versican und/oder Decorin und/oder Glypican bewirken mit UV-Lichtschutzfaktoren und/oder Antioxidantien führt durch die unterschiedlichen Mechanismen zu einer synergistischen Wirkungsweise und bietet einen hervorragenden Schutz gegen schädigende Einflüsse und Hautalterung durch UV-Lichteinwirkung.

### Lumican

Lumican gehört zur Familie der Keratansulfate, einer Leucin reichen Proteoglykangruppe (LRP), die in der Haut zusammen mit fibrillärem Collagen lokalisiert sind. In den Fibrillen verschiedener Bindegewebe trägt Lumican zu Elastizität und Stabilität des Gewebes bei. Histologisch erscheinen Collagenstränge in Lumican armem Gewebe desorganisiert und ohne Festigkeit. Insbesondere im Unterhautgewebe erkennt man einen Mangel an Ordnung und Orientierung von Collagensträngen und Fibroblasten. Die histologischen Anomalien können auch mit Elektronenmikroskopischen Untersuchungen anhand der Organisation der Collagenmatrix, erhöhten interfibrillären Räumen und veränderter Morphologie der Fibrillen verifiziert werden.

### Syndecan

Syndecane sind der Gruppe transmembraner Heparansulfat Proteoglykane zuzuordnen, die als Corezeptoren mit Integrin und Wachstumsfaktor Tyrosinkinase-Rezeptoren agieren. Durch ihre negative Oberflächenladung binden sie an positiv geladene Matrixabschnitte und bilden festsitzende Kontakte in der Basallamina. Syndecan-4 ist ein wichtiger Oberflächenrezeptor für die Wundheilung und die Angiogenese. Syndecan-1 ist das hauptsächliche Heparansulfat Proteoglykan der Epidermis. Es enthält Heparan mit Chondroitinsulfat Seitenketten und befindet sich in ausgereiftem, vollentwickeltem Gewebe vorwiegend in einfachen und schichtenförmigen Epithelien. In der Epidermis ist Syndecan-1 besonders in der Suprabasal Zellschicht, Lamina rara, zu finden. Es hat einen wesentlichen Anteil an der Migration und Proliferation von Keratinocyten bei der Wundheilung.

### Versican

Versican zählt zu den großen Chondroitinsulfat Proteoglykanen, die sich in der Lamina densa, dem mittleren Teil der Basalmembran und in der Lamina fibroreticularis befinden.

### Decorin

Decorin ist eines der kleinsten Proteoglykane aus der Leucinreichen Familie (LRP). Ein Dermatansulfat Proteoglykan, das im Wesentlichen in der extracellulären Matrix stark Collagenhaltiger Gewebe gefunden wird, wo es die Oberfläche von Collagenfibrillen bedeckt.

### Glypican

Glypican ist ein Proteoglykan, das über Glycosylphoshatidylinositol membrangebunden verankert ist und Antithrombin sowie Lipoprotein Lipasen auf der endothelialen Zelloberfläche bindet.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
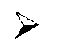 3-Benzylidencampher bzw. 3-Benzylidennorcampher und dessen Derivate, z.B. 3-(4-Methylbenzyliden)campher;
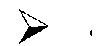 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
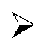 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
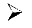 Ester der Salicylsäure, vorzugsweise Salicylsäure-2-ethylhexylester, Salicylsäure-4-isopropylbenzylester, Salicylsäurehomomenthylester;
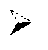 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
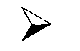 Ester der Benzalmalonsäure, vorzugsweise 4-Methoxybenzmalonsäuredi-2-ethylhexylester;
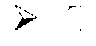 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
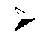 Propan-1,3-dione, wie z.B. 1-(4-ter-t.Butylphenyl)-3-(4'methoxyphenyl)propan-1,3-dion;
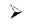 Ketotricyclo(5.2.1.0)decan-Derivate.

Als wasserlösliche Substanzen kommen in Frage:
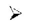 2-Phenylbenzimidazol-5-sulfonsäure und deren Alkali-, Erdalkali-, Ammonium-, Alkylammonium-, Alkanolammonium- und Glucammoniumsalze;
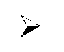 Sulfonsäurederivate von Benzophenonen, vorzugsweise 2-Hydroxy-4-methoxybenzophenon-5-sulfonsäure und ihre Salze;
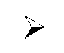 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Beispiele

Es wurden Glucosaminoglykane und Proteoglykane menschlicher Haut unterschiedlicher Altersstufen untersucht, um nachzuweisen, dass alterungsbedingte Änderungen von Proteoglykanen zum entsprechenden Aussehen der Haut und zu ihren mechanischen Eigenschaften beitragen.
Dazu wurden Proben von Kinderhaut, Erwachsenenhaut und gealterter Haut gewonnen und daraus Zellkulturen aus Keratinocyten und Fibroblasten kultiviert.

Um die Merkmale von menschlicher Haut unterschiedlichen Alters zu bestimmen, wurden die folgenden klassischen Techniken eingesetzt [19-WEGROWSKI Y, PALTOT V, GILLERY P, KALIS B, RANDOUX A, MAQUART FX ; Biochemical Journal, 1995, 307, 3, 673-678; WEGROWSKI Y, GILLERY P, KOTLARZ G , PERREAU C , GEORGES N and MAQUART FX, Molecular and Cellular Biochemistry, 2000, 205, 125-131].

### Für Glucosaminoglykane:

Verwendung von radioaktiv markierten Molekülen wie Tritium markierten Glucosaminen für alle Glukosaminoglykane außer Keratansulfat und ³⁵S für alle sulfatierten Glucosaminoglykane.
Quantitative Bestimmung der markierten Moleküle in Glucosaminoglykanen sowie Elektrophorese-Techniken

### Für Proteoglykane:

Northern Blot Technique zur Bestimmung der RNA-messengern in Zelle von Proteoglykanen mit geringen Molekulargewicht wie Lumican und Syndecan

### 1. Untersuchung humaner dermaler Fibroblasten von Spendern unterschiedlicher Alterskategorie

Die Menge an RNA-Messengern des Proteoglykans Lumican wurde in einer Kultur von Fibroblasten nach der Northern Blot Technique bestimmt und die Ergebnisse in Tabelle 1 als Verhältnis Menge RNA-Messenger für Lumican zur Menge an ¹⁸S ribosomaler RNA dargestellt.

| Tabelle 1: Verhältnis der Menge RNA-Messenger für Lumican zur Menge an ¹⁸S ribosomaler RNA für unterschiedliche Alterskategorien | | |
|---|---|---|
| Alter (Jahre) | Anzahl der Probanden | Mittelwert des Verhältnisses [Lumican RNAm / [18S ribosomal RNA] |
| 1-15 | 8 | 4,0 |
| 16-50 | 8 | 2,7 |
| 51 - 71 | 8 | 1,9 |

Die in Tabelle 1 ausgewiesenen Daten zeigen deutlich, dass die Menge an RNA-Messenger für Lumican mit zunehmendem Alter des Fibroblasten Spenders sinkt. Es ist daher zu erwarten, dass die Lumican Syntheserate in den Hautzellen einen wesentlichen Einfluß auf das Aussehen alternder Haut hat.

### 2. Untersuchung humaner Keratinocyten von Spendern unterschiedlicher Alterskategorie

Die Menge an RNA-Messengern des Proteoglykans Syndecan-1 wurde in einer Kultur von Keratinocyten nach der Northern Blot Technique bestimmt und die Ergebnisse in Tabelle 2 als Verhältnis Menge RNA-Messenger für Syndecan-1 zur Menge an ¹⁸S ribosomaler RNA dargestellt.

| Tabelle 2: Verhältnis der Menge RNA-Messenger für Syndecan-1 zur Menge an ¹⁸S ribosomaler RNA für unterschiedliche Alterskategorien | | |
|---|---|---|
| Alter (Jahre) | Anzahl der Probanden | Mittelwert des Verhältnisses [Syndecan-1 RNAm / [18S ribosomal RNA] |
| 1 - 15 | 4 | 5,1 |
| 16 - 50 | 4 | 3,2 |
| 51 - 71 | 4 | 3,1 |

Auch bei vergleichbaren Untersuchungen am Proteoglykan Syndecan-1 erkennt man eine Abnahme der RNA-Messenger mit zunehmendem Alter der Keratinocyten-Spender, so dass auch bei diesem Proteoglykan angenommen werden muß. Dass die Syntheserate einen wichtigen Einfluß auf die Hautalterung hat.

## Patentansprüche

1. Verfahren zur kosmetischen Behandlung zur Verbesserung und/oder zum Schutz der menschlichen Haut, Kopfhaut und/oder Schleimhaut, **dadurch gekennzeichnet, dass** man eine Zubereitung, enthaltend mindestens eine Substanz, welche eine Modulation von Proteoglykanen bewirkt, topisch aufträgt.

2. Verfahren zur kosmetischen Behandlung zur Verbesserung und/oder zum Schutz der menschlichen Haut, Kopfhaut und/oder Schleimhaut, **dadurch gekennzeichnet, dass** man eine Zubereitung, enthaltend mindestens eine Substanz, welche eine Modulation von Lumican und/oder Syndecan und/oder Versican und/oder Decorin und/oder Glypican bewirkt, topisch aufträgt

3. Verwendung einer Substanz, welche eine Modulation von Lumican und/oder Syndecan und/oder Versican und/oder Decorin und/oder Glypican bewirkt, zur Herstellung kosmetischer Mittel zum Schutz der menschlichen Haut, Kopfhaut und/oder Schleimhaut gegen Alterung.

4. Verwendung einer Substanz, welche eine Modulation von Lumican und/oder Syndecan und/oder Versican und/oder Decorin und/oder Glypican bewirkt, zur Herstellung kosmetischer Mittel zum Schutz der menschlichen Haut, Kopfhaut und/oder Schleimhaut gegen toxische Umwelteinflüsse.

5. Verwendung einer Substanz, welche eine Modulation von Lumican und/oder Syndecan und/oder Versican und/oder Decorin und/oder Glypican bewirkt, zur Herstellung kosmetischer Mittel zum Schutz der menschlichen Haut, Kopfhaut und/oder Schleimhaut gegen Schädigungen durch UV-Lichteinwirkung.

6. Verwendung einer Substanz, welche eine Modulation von Lumican und/oder Syndecan und/oder Versican und/oder Decorin und/oder Glypican bewirkt, zur Herstellung kosmetischer Mittel zum Schutz der menschlichen Haut, Kopfhaut und/oder Schleimhaut gegen oxidativen Stress.

7. Verwendung einer Substanz, welche eine Modulation von Lumican und/oder Syndecan und/oder Versican und/oder Decorin und/oder Glypican bewirkt, zur Herstellung von dermopharmazeutischen Zubereitungen zur Verbesserung der Wundheilung.

8. Verwendung einer Substanz, welche eine Modulation von Lumican und/oder Syndecan und/oder Versican und/oder Decorin und/oder Glypican bewirkt, zur Herstellung kosmetischer Mittel zur Verbesserung der Funktionen der Dermal-Epidermal-Junctions.

9. Verwendung einer Substanz, welche eine Modulation von Lumican und/oder Syndecan und/oder Versican und/oder Decorin und/oder Glypican bewirkt, zur Herstellung von dermopharmazeutischen Zubereitungen gegen Alopecie, Cellulitis oder Rosacea.

10. Verwendung gemäß mindestens einem der Ansprüche 3 bis 9, **dadurch gekennzeichnet, dass** die Substanz, welche eine Modulation von Lumican und/oder Syndecan und/oder Versican und/oder Decorin und/oder Glypican bewirkt, ein Pflanzenextrakt und/oder ein Extrakt aus Mikroorganismen und/oder ein Fermentationsprodukt pflanzlicher Herkunft ist.

11. Verwendung gemäß Anspruch 10, **dadurch gekennzeichnet, dass** der Pflanzenextrakt aus der Pflanze Pisum sativum und/oder Vigna aconitifolia gewonnen ist.

12. Verwendung gemäß mindestens einem der Ansprüche 3 bis 9, **dadurch gekennzeichnet, dass** die Substanz, welche eine Modulation von Lumican und/oder Syndecan und/oder Versican und/oder Decorin und/oder Glypican bewirkt, Mannitol und/oder Cyclodextrin und/oder Hefeextrakt und/oder Dinatriumsuccinat enthält.

13. Verwendung gemäß mindestens einem der Ansprüche 3 bis 9, **dadurch gekennzeichnet, dass** die Substanz, welche eine Modulation von Lumican und/oder Syndecan und/oder Versican und/oder Decorin und/oder Glypican bewirkt, ausgewählt ist aus der Gruppe, die gebildet wird von Phytosterolen, Phytoestrogenen, Triterpenen, Triterpensaponinen und Steroidsaponinen, Peptiden und Flavonoiden, sowie Flavonoidderivaten.

14. Kosmetische Zubereitungen, enthaltend mindestens eine Substanz, welche eine Modulation von Lumican und/oder Syndecan und/oder Versican und/oder Decorin und/oder Glypican bewirkt, und UV-Lichtschutzfaktoren und/oder Antioxidantien.
